# EUROPÄISCHE PATENTSCHRIFT

(11) **EP 1 140 261 B1**
(45) Veröffentlichungstag und Bekanntmachung des Hinweises auf die Patenterteilung: **04.06.2003**
(21) Anmeldenummer: 99955638.4
(22) Anmeldetag: 01.12.1999
(51) Int. Cl.: A61M 5/50

(54) **INJEKTIONSSPRITZE**
INJECTING SYRINGE
SERINGUE D'INJECTION

(30) Priorität: 08.01.1999 CH 3099; 05.11.1999 CH 203099
(43) Veröffentlichungstag der Anmeldung: 10.10.2001
(73) Patentinhaber: Schöttli, Theodor, 8253 Diessenhofen (CH)
(72) Erfinder: Schöttli, Theodor, 8253 Diessenhofen (CH)
(74) Vertreter: Gachnang, Hans Rudolf
(86) Internationale Anmeldenummer: CH9900576
(87) Internationale Veröffentlichungsnummer: WO00040284

(56) Entgegenhaltungen:
- EP-A- 0 409 134
- US-A- 4 863 427
- US-A- 4 950 240
- US-A- 5 000 735
- US-A- 5 163 908

## Beschreibung

Gegenstand der Erfindung ist eine Injektionsspritze gemäss Oberbegriff des Patentanspruchs 1.

Injektionsspritzen sind in vielen Ausführungen bekannt. Sie umfassen einen Spritzenzylinder mit einer Kupplungsvorrichtung für eine Injektionsnadel und einen Kolben mit einem Kolbenkopf und einem Kolbenschaft sowie einen Fingerflansch zum Betätigen des Kolbens.
Solche Injektionsspritzen werden nicht ausschliesslich durch ausgebildetes Personal verwendet, sondern dienen vermehrt zur Injektion von Betäubungsmitteln und gelangen dadurch in die Hände von nicht ausgebildeten Personen, welche bezüglich Sterilität keine Sorgfalt walten lassen.

Es sind aus diesem Grunde bereits Injektionsspritzen bekannt geworden, mit denen der Erstgebrauch angezeigt werden soll bzw. die nur ein einziges Mal benutzbar sein sollen. Die Möglichkeit, die Spritze nur ein einziges Mal zu benutzen, schützt nebst Rauschmittelsüchtigen auch andere Kranke vor allem in unterentwickelten Ländern davor, in Kontakt mit verseuchtem Blut an einer bereits benutzten Spritze zu gelangen.

Aus der EP-0438453 ist eine Injektionsspritze bekannt, deren Kolbenkopf eine axiale Bohrung aufweist, die kolbenbodenseitig durch eine Scheibe mit umlaufender Sollbruchstelle verschlossen ist. Alternativ zu einer Scheibe mit Sollbruchstelle ist es aus derselben Schrift auch bekannt, in den Kolbenboden einen Ring mit zentraler Sollbruchstelle einzusetzen. An der Rückseite des Spritzenkonus, auf den die Injektionsnadel aufgesetzt werden kann, ist eine in den Hubraum hineinragende Schneide ausgebildet, die bei vollständigem Entleeren der Spritze, das heißt bei vollständigem Vorschieben des Kolbenkopfs, die Scheibe entlang der Sollbruchstelle vom Kolbenboden abtrennt. Das Wiedereinziehen eines Medikaments wird dann verhindert, weil wegen des Durchbruchs des Kolbenbodens kein Vakuum innerhalb der Injektionsspritze mehr aufgebaut werden kann.

Diese bekannte Injektionsspritze weist den Nachteil auf, dass zum Durchbrechen des Kolbenbodens ein fühlbarer Widerstand überwunden werden muss. Geschickte Süchtige oder geschicktes Medizinalpersonal in Spitälern in unterentwickelten Ländern sind deshalb ohne weiteres in der Lage den Inhalt der Injektionsspritze nur soweit zu entleeren, dass eine Zerstörung bzw. eine Perforation des Kolbenbodens nicht erfolgt. Die Spritze kann folglich beliebig oft wieder benutzt werden.

Aus der WO 97/31665 ist bereits ein zweiteiliger Kolben bekannt, dessen beide Teile durch eine zerbrechbare Brücke miteinander verbunden sind und welcher bei Injektionsspritzen eingesetzt werden kann.

Die Aufgabe der vorliegenden Erfindung besteht nun darin, eine Injektionsspritze nach dem Oberbegriff des Patentanspruchs 1 derart weiterzubilden, dass nach einmaligem Gebrauch ein erneutes Einziehen von Flüssigkeit unmöglich ist. Gelöst wird diese Aufgabe durch eine Injektionsspritze mit den Merkmalen des Patentanspruchs 1. Vorteilhafte Ausgestaltungen der Erfindung sind in den abhängigen Ansprüchen definiert.

Der nach der Erfindung ausgebildete Kolbenkopf bricht während der Injektion oder spätestens am Ende des Injektionsvorgangs von der Kolbenstange ab und lässt sich nicht mehr zurückziehen. Er verbleibt im Spritzenzylinder stecken.
In der Ausgestaltung der erfindungsgemässen Spritze mit Stegen, die in einer Mantelfläche eines Kegelstumpfs liegen, kann die Bruchlast in Abhängigkeit der Verschieberichtung des Spritzenkolbens unterschiedlich gross gestaltet werden. Dies ermöglicht das Aufziehen der Flüssigkeit, ohne Gefahr zu laufen, dass die Stege brechen. Bei der nachfolgenden Injektion hingegen brechen die Stege bereits bei wenigen beispielsweise 7-10 Newton Schubkraft. Dies beeinträchtigt die Funktion der Spritze nicht, weil der Spritzeninhalt dennoch ausgestossen werden kann. Das Schaftende schiebt den Kolbenkopf in jedem Fall nach vorne. Das Schaftende liegt dann an der das Ende der Bohrung überspannenden Dichtungskappe an.
Die Herstellung der Spritze, d.h. sowohl des Zylinders und des Kolbens und, falls der Kolben eine Gummidichtung aufweist, auch der Gummikappe ist kostengünstig, da nur zwei oder drei Teile gefertigt und montiert werden müssen.

Anhand illustrierter Ausführungsbeispiele wird die Erfindung näher erläutert. Es zeigen
- Figur 1: eine perspektivische Darstellung einer Einwegspritze mit teilweise eingeschobenem Kolbenkopf,
- Figur 2: einen Längsschnitt durch eine Spritze mit in Ausgangsstellung befindlichem Kolbenkopf,
- Figur 3: einen Längsschnitt durch die Spritze mit vollständig vorgeschobenem Kolbenkopf, der vom Schaft abgetrennt ist,
- Figur 4: einen Querschnitt durch den Kolbenkopf und das Schaftende längs Linie IV -IV in Fig. 5,
- Figur 5: einen Längsschnitt durch den Kolbenkopf und das Schaftende,
- Figur 6: einen Längsschnitt durch den Kolbenkopf in einer weiteren Ausgestaltung der Erfindung,
- Figur 7: einen Längsschnitt durch den Kolbenkopf in einer weiteren Ausgestaltung der Erfindung,
- Figur 8: einen Längsschnitt durch eine Spritze mit in Ausgangsstellung befindlichem Kolbenkopf ohne zusätzliche Dichtungskappe,
- Figur 9: eine Ansicht eines Kolbens einer weiteren Ausführungsform der Erfindung.

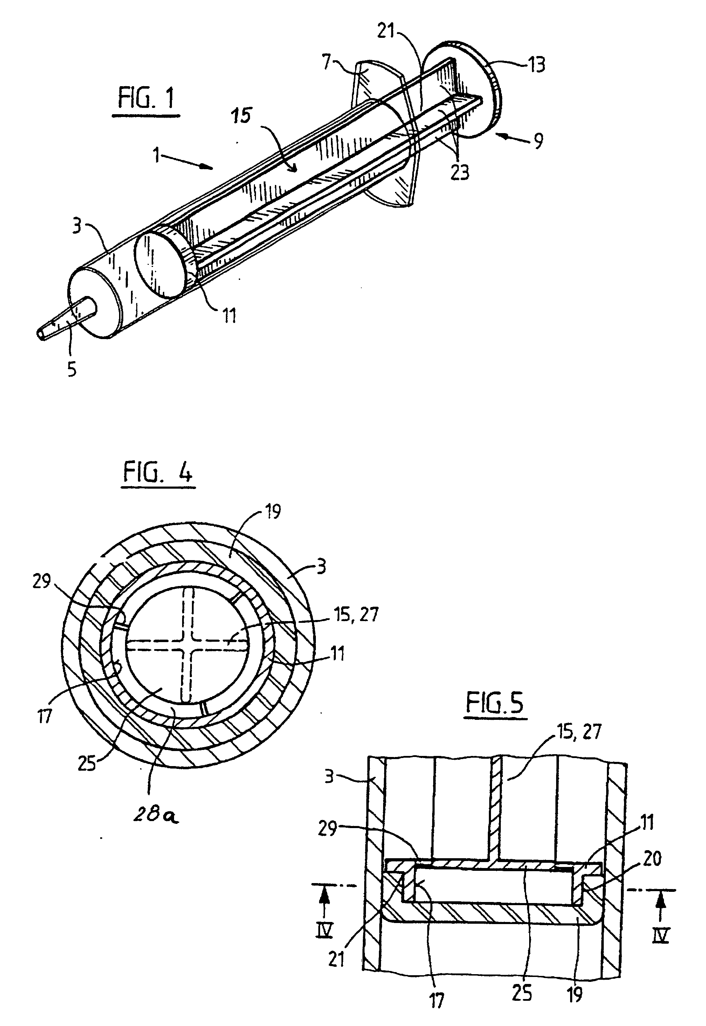

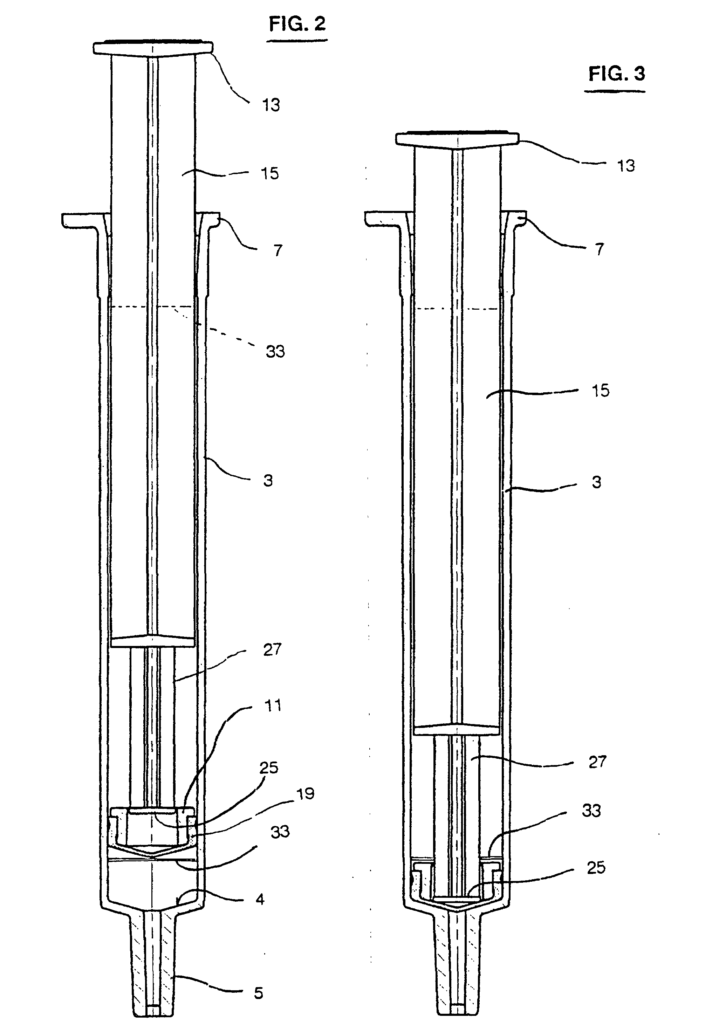

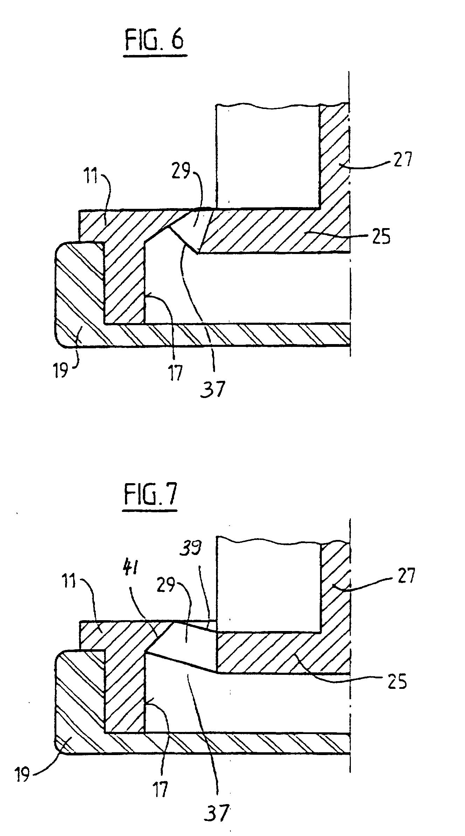

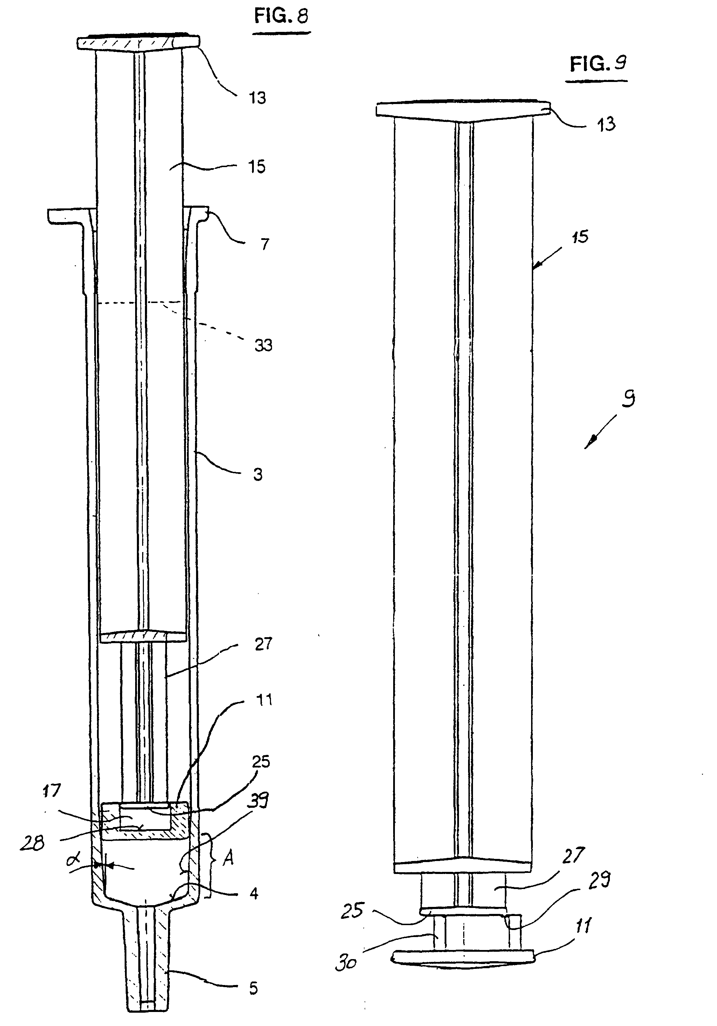

In der perspektivischen Übersichtsdarstellung gemäss Figur 1, ist mit Bezugszeichen 1 eine Einwegspritze bezeichnet. Diese umfasst einen Spritzenzylinder 3 mit einer Kupplungsvorrichtung für eine Injektionsnadel (nicht aufgesetzt), z.B. einen Nadelkonus 5, sowie zwei radial abstehende Griffplatten 7. Im Spritzenzylinder 3 ist ein teilweise vorgeschobener Spritzenkolben 9 dargestellt, an dessen vorderem Schaftende ein Kolbenkopf 11 aufgesetzt und an dessen hinterem Schaftende ein Fingerflansch 13 ausgebildet sind. Zwischen dem Kolbenkopf 11 und dem Fingerflansch 13 liegt ein beispielsweise einen kreuzförmigen Querschnitt aufweisender Schaft 15. Die beiden Hauptteile, Spritzenzylinder 3 und Spritzenkolben 9, sind aus Kunststoff durch Spritzen hergestellt. Im Kolbenkopf 11 ist eine axial verlaufende, den Kolbenkopf 11 ganz (vergl. Fig. 2 bis 7) oder nur teilweise (Fig. 8 und 9) durchdringende Bohrung 17 eingelassen. Eine vorzugsweise kappenförmige Dichtung 19 umgibt in der ersten Ausführungsform den Kolbenkopf 11 peripher und seine stirnseitige Bodenfläche. Die kappenförmige Dichtung 19 erstreckt sich über mindestens einen Teil der Peripherie 20 des Dichtungskolbens 11 und liegt dort innerhalb eines umlaufenden Absatzes 21 eingebettet (vgl. Figur 5). Der kreuzförmige Schaft 15 weist mindestens im vorderen Abschnitt einen Durchmesser auf, der kleiner ist als der Durchmesser der Bohrung 17.
In einer Ausgestaltung der Erfindung sitzt auf dem Ende des Schafts 15 eine Scheibe 25, deren Durchmesser gleich oder grösser ist als der Durchmesser der Balken 23 des Schafts 15, jedoch kleiner ist als der Durchmesser der Bohrung 17.

Zwischen der Peripherie der Scheibe 25 bzw. dem vorderen, vorzugsweise eine geringere Dicke aufweisenden Schaftabschnitt 27 und der Bohrung 17 ergibt sich ein 28 a spalt.

Die Verbindung zwischen dem Schaftabschnitt 27 bzw. der Scheibe 25 und dem Kolbenkopf 11 erfolgt durch feine faden-, stäbchen- oder filmförmige Stege 29, welche sich von dem Balken 23 am Schaftabschnitt 27 oder von der Peripherie der Scheibe 25 radial an die Wandung der Bohrung 17 erstrecken und mit dieser verbunden sind. Die Verbindung der im Querschnitt runden oder eckigen Stege 29 kann am Ende der Bohrung 17 oder axial versetzt im Innern der letzteren erfolgen. Die Festigkeit der Stege 29 ist derart bemessen, dass sie bei Überschreiten einer vorgebbaren Axialkraft, z.B. 10N vom Schaft 15 oder der Scheibe 25 abgeschert werden. Eine nähere Erläuterung dieses Vorgangs erfolgt bei der Funktionsbeschreibung.

Im Hubraum des Spritzenzylinders 3 kann in einem Abstand vom Spritzenzylinderboden, der grösser ist als die axiale Ausdehnung des Kolbenkopfs 11 ein umlaufender Wulst 33 ausgebildet sein, welcher in den Hubraum des Zylinders 3 und damit in den Verschiebeweg des Kolbenkopfs 11 hineinragt und dessen Querschnitt verkleinert. Die radiale Ausdehnung des Wulstes 33 ist sehr gering und hängt vom Durchmesser des Spritzenzylinders 3 ab.

In der Ausgestaltung der Erfindung nach den Figuren 6 und 7 weisen die Stege 29 einen viereckigen Querschnitt auf, wobei die erste, dem Kolbenkopfboden zugewendete Kante 37 der Stege geneigt verläuft. Die dem Kolbenkopfboden abgewendete zweite Kante 39 kann parallel zum Kolbenkopfboden oder parallel zur ersten Kante 37 verlaufen. Die Anbindungsbereiche der Stege 29 am Kolbenkopf 11 und am Schaftabschnitt 27 oder an der auf dem Schaftabschnitt 27 sitzenden Scheibe 25 können parallel verlaufen oder - wie in den Beispielen in den Figuren 6 und 7 dargestellt - kann die Bohrung 17 im Bereich der Stege 29 einen konisch verlaufenden Abschnitt 39 aufweisen. Analog kann auch die Peripherie der Scheibe 25 konisch verlaufend ausgebildet sein, d.h. die Scheibe 25 weist die Gestalt eines Kegelstumpfs auf (Fig.6).

In der Ausgestaltung der Erfindung gemäss Figur 8 ist am Kolbenkopf 11 keine zusätzliche elastische Dichtung angebracht, sondern der Kolbenkopf 11 liegt mit seiner Peripherie direkt auf der Wandung des Spritzenzylinders 3 an. Der Schaftabschnitt 27 ist wiederum mittels feinen Stegen 29 mit dem Kolbenkopf 11 verbunden, wobei im Kolbenkopf 11 eine Sackbohrung 17 ausgebildet ist, in welche der Schaftabschnitt 27 nach dem Brechen der Stege 29 einfahren und am Boden 28 anstossen kann.
In der Ausgestaltung der Erfindung nach Figur 9 ist am Schaftende wiederum ein Kolbenkopf 11 dargestellt, der direkt mit seiner Peripherie an der Wandung des Spritzenzylinders dichtend anliegt. Die Verbindung zwischen dem Kolbenkopf 11, der hier scheibenförmig ausgebildet ist, und dem Schaftabschnitt 27 erfolgt durch beispielsweise drei Füsse 30, die einseitig fest mit dem scheibenförmigen Teil des Dichtungskopfs 11 verbunden sind und auf der anderen Seite je durch einen Steg 29 mit dem vorderen Ende des Schaftabschnitts 27 bzw. mit der Scheibe 25. Vorzugsweise entspricht die Länge der Füsse 23 dem Abstand der Scheibe 25 vom unteren Ende des oberen Teils des Schafts 15.

Im folgenden wird die Funktionsweise der Einwegspritze 1 näher erläutert.
Beim Hersteller der Spritze 1 wird nach dem Spritzen der Einzelteile bei der dreiteiligen Ausführung die Dichtung 19 auf den Kolbenkopf 11 aufgestülpt und anschliessend der Spritzenkolben 9 in den Spritzenzylinder 3 eingeschoben, und zwar nur soweit, damit die Dichtung 19 noch nicht in den Wulst 33 hineinragt, d.h. sich die beiden Teile nicht berühren. Das Einschieben des Spritzenkolbens 9 in den Spritzenzylinder 3 erfolgt üblicherweise durch ein Montage- oder Handling-Gerät, so dass die Lage des Spritzenkolbens 9 sehr genau eingestellt werden kann.
In der Ausgestaltung der Erfindung nach den Figuren 8 und 9, die ohne Dichtungskappe 19 auskommen, wird nach der Herstellung des Spritzenkolbens 9 letzterer direkt in den Spritzenzylinder 3 eingeschoben. Das Einschieben erfolgt allerdings nur bis das vordere Ende des Kolbenkopfs den Anfang eines konischen Bereichs A (siehe Figur 8) erreicht hat, sofern der Spritzenzylinder 3 mit einem solchen einen Konuswinkel alpha aufweisenden Bereich A ausgerüstet ist. Bei einem durchgehend zylindrischen Spritzenzylinder 3 kann der Kolben 9 bis zum Kolbenboden 4 vorgeschoben werden.

Der Benutzer der Spritze 1 füllt diese in herkömmlicher Weise, indem er den Spritzenkolben 7 zurückzieht. Nach der Entlüftung durch Ausblasen der Luft im vorderen Teil des Spritzenzylinders 3 erfolgt die Injektion, wobei der Kolbenkopf 11 zur vollständigen Entleerung bis zum Anschlag am Boden 4 am nadelseitigen Ende des Spritzenzylinders 3 vorgeschoben wird. Auf den letzten Millimetern des Vorschubs beim Überfahren des Wulstes 33, falls ein solcher vorgesehen ist, oder beim Einfahren in den konischen Bereich A und/oder beim Auspressen des letzten Rests der Flüssigkeit brechen die Stege 29 wegen des erhöhten Widerstands und der Schaft 15 gleitet in der Bohrung 17 bzw. zwischen den Füssen 30 (Fig. 9) nach vorn und stösst am Grund 28 der Bohrung 17 bzw. die Füsse 30 am Schaftende an.
Der Kolbenkopf 11 lässt sich so sicher bis ans Hubraumende am Boden 4 des Spritzenzylinders 3 vorschieben, obwohl die Stege 29 gebrochen sind.
Ein erneutes Aufziehen von Flüssigkeit ist ausgeschlossen. Die Einwegspritze 1 ist damit unbrauchbar geworden.

## Patentansprüche

1. Injektionsspritze (1) mit einem Spritzenzylinder (3) mit einer am vorderen Zylinderende ausgebildeten Kupplungsvorrichtung (5) zum Aufsetzen einer Injektionsnadel, einem am hinteren Zylinderende ausgebildeten Griffplattenpaar (7), einem im Hubraum des Spritzenzylinders (3) längsführbaren Spritzenkolben (9), umfassend einen einen vorderen Schaftabschnitt (27) aufweisenden Schaft (15) mit einem Kolbenkopf (11) mit peripher umlaufender Dichtungsfläche (19) und einem Fingerflansch (13), wobei der Kolbenkopf (11) mit wenigstens einem Steg (29) am Schaft (15) angebunden ist, dessen Festigkeit derart bemessen ist, dass er bei Überschreiten einer vorgebbaren Axialkraft, wie sie während der Injektion oder spätestens am Ende des Injektionsvorganges auftritt, bricht, und wobei am Kolbenkopf (11) Mittel (28) zum Abstützen des Schaftendes nach Bruch des Steges (29) ausgebildet sind,
**dadurch gekennzeichnet, dass**
- im Kolbenkopf (11) eine axial verlaufende Bohrung (17) ausgebildet ist,
- dass der Schaftabschnitt (27) am kolbenkopfseitigen Ende einen Querschnitt aufweist, der kleiner ist als der Querschnitt der Bohrung (17) am schaftseitigen Ende, derart, dass zwischen diesem Ende und der Bohrung (17) ein Spalt (28a) vorliegt,
- dass das kolbenkopfseitige Ende des Schaftabschnitts (27) durch eine Mehrzehl von film-, Stäbchen- oder fadenförmigen Stegen (29) mit dem Kolbenkopf (11) verbunden ist, und
- dass der kolbenkoph (11) und der Schaftabschnitts (27) einstückig ausgelildet sind.

2. Injektionsspritze nach Anspruch 1,
**dadurch gekennzeichnet,**
**dass** die Stege (29) im wesentlichen radial verlaufend zwischen dem Schaft (15) und dem Kolbenkopf (11) angeordnet sind.

3. Injektionsspritze nach einem der Ansprüche 1 oder 2, **dadurch gekennzeichnet,**
**dass** auf dem kolbenkopfseitigen Ende des Schaftes (15) eine Scheibe (25) ausgebildet oder aufgesetzt ist, deren Durchmesser gleich oder größer ist als der Durchmesser der Balken (23) des Schaftes (15), jedoch kleiner ist als der Durchmesser der Bohrung (17) und dass die Stege (29) an dieser Scheibe (25) befestigt sind.

4. Injektionsspritze nach Anspruch 3,
**dadurch gekennzeichnet,**
**dass** die Kanten (37, 39) der Stege (29) sich mit zunehmendem radialen Abstand vom Schaft (15) oder der Scheibe (25) nähern und die Kontaktfläche mit dem Kolbenkopf (11) kleiner ist als mit der Scheibe (25) oder dass die Stege (29) in einer kegelstumpfförmigen Fläche verlaufend ausgebildet sind.

5. Injektionsspritze nach einem der Ansprüche 1 bis 4,
**dadurch gekennzeichnet,**
**dass** die Stege (29) am schaftseitigen Ende der Bohrung (17) oder axial beabstandet vom schaftseitigen Ende im Innern der Bohrung (17) mit dem Kolbenkopf (11) verbunden sind.

6. Injektionsspritze nach einem der Ansprüche 1 bis 3,
**dadurch gekennzeichnet,**
**dass** die Bohrung (17) und/oder die Peripherie der Scheibe (25) an der Anbindestelle der Stege (29) konisch verlaufend ausgebildet ist.

7. Injektionsspritze nach einem der Ansprüche 1 bis 4,
**dadurch gekennzeichnet,**
**dass** die Dichtung (19) axial mindestens über einen Teil der Peripherie des Dichtungskolbens (11) verläuft und dass die Dichtung (19) kappenförmig ausgebildet ist.

8. Injektionsspritze nach einem der Ansprüche 1 bis 7,
**dadurch gekennzeichnet,**
**dass** in einem Abstand zum vorderen und/oder hinteren Ende des Hubraums im Spritzenzylinder (3) je ein ganz oder teilweise umlaufender, den Querschnitt verengender Wulst (33) und/oder am vorderen Ende ein konisch sich verengender Abschnitt (A) ausgebildet ist.

9. Injektionsspritze (1) mit einem Spritzenzylinder (3) mit einer am vorderen Zylinderende ausgebildeten Kupplungsvorrichtung (5) zum Aufsetzen einer Injektionsnadel, einem am hinteren Zylinderende ausgebildeten Griffplattenpaar (7), einem im Hubraum des Spritzenzylinders (3) längsführbaren Spritzenkolben (9), umfassend einen Schaft (15) mit einem Kolbenkopf (11) mit peripher umlaufender Dichtungsfläche (19) und einem Fingerflansch (13), wobei der Kolbenkopf (11) mit wenigstens einem Steg (29) am Schaft (15) angebunden ist, dessen Festigkeit derart bemessen ist, dass er bei Überschreiten einer vorgebbaren Axialkraft, wie sie während der Injektion oder spätestens am Ende des Injektionsvorganges auftritt, bricht, und wobei am Kolbenkopt (11) Mittel (30) zum Abstützen des Schaftendes nach Bruch des Steges (29) ausgebildet sind,
**dadurch gekennzeichnet,**
**dass** am Kolbenkopf (11) zum Schaft (15) weisende Stützfüße (30) angeformt sind, zwischen denen der Schaft (15) bis zum Anstoßen der Stützfüße (30) am Schaftende gleiten kann und an deren schaftseitigen Enden eine Mehrzehl von film-, stäbchen- oder fadenförmigen Stegen (29) angebunden ist, welche die Verbindung zum Schaft (15) herstellt, und dass der Kolbenkopf (11) und der schaft (15) einstückig ausgelildet sind.

10. Injektionsspritze nach Anspruch 9,
**dadurch gekennzeichnet,**
**dass** der Kolbenkopf (11) scheibenförmig ausgebildet ist.

## Claims

1. Syringe (1) including an injecting cylinder (3) having a coupling device (5), which is configured on the front end portion of the cylinder for the accommodation of an injection needle, two gripping plates (7) configured on the rear end portion of the cylinder, an injection plunger (9), which is longitudinally displaceable within the piston travel of the injecting cylinder (3), including a shank (15), which has a front shank portion (27), said shank (15) having a piston head (11) with peripherally circumferential sealing face (19) and a finger flange (13), the piston head (11) being attached to the shank (15) by at least one web (29), the strength of which web is dimensioned in such a manner that it breaks when a predetermined axial force is exceeded, as occurs during the injection or, at the latest, at the end of the injection process, and means (28) being configured on the piston head (11) to support the end of the shank once the web (29) has broken, **characterised in that**
- an axially extending bore (17) is configured in the piston head (11),
- **in that** the shank portion (27) at the piston head side face has a cross-section which is smaller than the cross-section of the bore (17) on the shank side face, such that between this face and the bore (17) there is a gap (28a),
- **in that** the piston head side face of the shank portion (27) is connected to the piston head (11) through film-like, rod-like or thread-like webs (29), and
- **in that** the piston head (11) and the shank portion (27) are integrally formed.

2. Syringe according to claim 1, **characterised in that** the webs (29) are disposed substantially so as to extend radially between the shank (15) and the piston head (11).

3. Syringe according to one of claims 1 or 2, **characterised in that** a disc (25) is configured or mounted on the piston head side face of the shank (15), the diameter of which disc (25) is equal to or in excess of the diameter of the bar (23) of the shank (15), but is smaller than the diameter of the bore (17) and **in that** the webs (29) are secured to this disc.

4. Syringe according to claim 3, **characterised in that** the edges (37, 39) of the webs (29) converge at an increasing radial distance from the shank (15) or the disc (25) and the contact area with the piston head (11) is smaller than the contact area with the disc (25), or **in that** the webs (29) are configured extending in a truncated cone-like area.

5. Syringe according to one of claims 1 to 4, **characterised in that** the webs (29) are connected to the piston head (11) at the shank-side face of the bore (17) or are connected to the piston head (11) in the interior of the bore (17) at an axial spacing from the shank side face.

6. Syringe according to one of claims 1 or 3, **characterised in that** the bore (17) and/or the periphery of the disc (25) is configured so as to extend in a conical manner at the point of attachment of the webs (29).

7. Syringe according to one of claims 1 to 4, **characterised in that** the seal (19) extends axially at least over a portion of the periphery of the sealing piston (11) and **in that** the seal (19) is configured so as to be cap-like.

8. Syringe according to one of claims 1 to 7, **characterised in that** at a spacing from the front and/or rear face of the piston travel in the injection cylinder (3) there is a beading (33), which extends respectively the entire way or partially round and constricts the cross-section, and/or there is a conically narrowing portion (A) at the front face.

9. Syringe (1) including an injection cylinder (3) having a coupling device (5) configured at the front end portion of the cylinder for the accommodation of an injection needle, two gripping plates (7) configured at the rear end portion of the cylinder, an injection plunger (9), which is longitudinally displaceable within the piston travel of the injection cylinder (3), including a shank (15) with a piston head (11) with peripherally circumferential seal face (19) and a finger flange (13), the piston head (11) being attached to the shank (15) by at least one web (29), the strength of which is dimensioned in such a manner that it breaks when a predetermined axial force is exceeded, as occurs during the injection or, at the latest, at the end of the injection procedure, and means (30) being configured on the piston head (11) to support the end of the shank once the web (29) has broken, **characterised in that** a plurality of supporting feet (30) facing the shank (15) are integrally formed on the piston head (11), between which supporting feet (30) the shank (15) can slide until abutting the supporting feet (30) at the shank end and on the shank-side faces of which supporting feet (30) film-like, rod-like or thread-like webs (29) are attached, which webs (29) produce the connection to the shank (15) and **in that** the piston head (11) and the shank (15) are integrally formed.

10. Syringe according to claim 9, **characterised in that** the piston head (11) is configured so as to be disc-like.

## Revendications

1. Seringue d'injection (1), avec un cylindre de seringue (3) doté d'un dispositif d'accouplement (5) configuré à l'extrémité avant du cylindre et destiné à la mise en place d'une aiguille d'injection, avec une paire de plaques de préhension (7) configurée à l'extrémité arrière du cylindre, avec un piston d'injection (9) pouvant être guidé longitudinalement à l'intérieur du cylindre de seringue (3), comprenant un corps (15) présentant une partie avant de corps (27) et doté d'une tête de piston (11) à face d'étanchéité (19) périphériquement entourante et d'un collet (13) pour appliquer un doigt, la tête de piston (11) étant rattachée au corps (15) par au moins une nervure (29) dont la résistance est prévue de telle sorte qu'elle se rompt lors du dépassement d'une force axiale prédéfinissable, telle qu'elle apparaît pendant l'injection ou au plus tard à la fin de l'opération d'injection, et des moyens (28) étant configurés sur la tête de piston (11) pour soutenir l'extrémité du corps à la suite de la rupture de la nervure (29),
**caractérisée**
- **en ce qu'**un perçage (17) s'étendant axialement est configuré dans la tête de piston (11),
- **en ce que** la partie de corps (27) présente à l'extrémité côté tête de piston une section qui est plus petite que la section du perçage (17) à l'extrémité côté corps, de telle sorte qu'une fente (28a) est présente entre cette extrémité et le perçage (17),
- **en ce que** l'extrémité côté tête de piston de la partie de corps (27) est reliée à la tête de piston (11) par une pluralité de nervures (29) pelliculaires ou filiformes ou en forme de baguettes,
- et **en ce que** la tête de piston (11) et la partie de corps (27) sont réalisées d'un seul tenant.

2. Seringue d'injection selon la revendication 1, **caractérisée en ce que** les nervures (29) sont disposées en s'étendant essentiellement radialement entre le corps (15) et la tête de piston (11).

3. Seringue d'injection selon la revendication 1 ou 2, **caractérisée en ce qu'**un disque (25) est configuré ou monté sur l'extrémité côté tête de piston du corps (15), disque dont le diamètre est supérieur ou égal au diamètre des barrettes (23) du corps (15), mais inférieur au diamètre du perçage (17), et **en ce que** les nervures (29) sont fixées sur ce disque (25).

4. Seringue d'injection selon la revendication 3, **caractérisée en ce que** les bords (37, 39) des nervures (29) se rapprochent avec augmentation de la distance radiale du corps (15) ou du disque (25), et la surface de contact est plus petite avec la tête de piston (11) qu'avec le disque (25), ou **en ce que** les nervures (29) sont configurées en s'étendant dans une surface tronconique.

5. Seringue d'injection selon l'une des revendications 1 à 4, **caractérisée en ce que** les nervures (29) sont reliées à la tête de piston (11) à l'extrémité côté corps du perçage (17) ou, à distance axiale de l'extrémité côté corps, à l'intérieur du perçage (17).

6. Seringue d'injection selon l'une des revendications 1 à 3, **caractérisée en ce que** le perçage (17) et/ou la périphérie du disque (25) sont réalisés à allure conique au point de rattachement des nervures (29).

7. Seringue d'injection selon l'une des revendications 1 à 4, **caractérisée en ce que** le joint d'étanchéité (19) s'étend axialement au moins sur une partie de la périphérie du piston d'étanchéité (11), et **en ce que** le joint d'étanchéité (19) est réalisé en forme de calotte.

8. Seringue d'injection selon l'une des revendications 1 à 7, **caractérisée en ce qu'**un bourrelet (33) respectif, partiellement ou totalement entourant et rétrécissant la section, est configuré à une certaine distance de l'extrémité avant et/ou de l'extrémité arrière de l'espace pour le piston dans le cylindre de seringue (3), et/ou une partie (A) se rétrécissant coniquement est configurée à l'extrémité avant.

9. Seringue d'injection (1), avec un cylindre de seringue (3) doté d'un dispositif d'accouplement (5) configuré à l'extrémité avant du cylindre et destiné à la mise en place d'une aiguille d'injection, avec une paire de plaques de préhension (7) configurée à l'extrémité arrière du cylindre, avec un piston d'injection (9) pouvant être guidé longitudinalement à l'intérieur du cylindre de seringue (3), comprenant un corps (15) doté d'une tête de piston (11) à face d'étanchéité (19) périphériquement entourante et d'un collet (13) pour appliquer un doigt, la tête de piston (11) étant rattachée au corps (15) par au moins une nervure (29) dont la résistance est prévue de telle sorte qu'elle se rompt lors du dépassement d'une force axiale prédéfinissable, telle qu'elle apparaît pendant l'injection ou au plus tard à la fin de l'opération d'injection, et des moyens (30) étant configurés sur la tête de piston (11) pour soutenir l'extrémité du corps à la suite de la rupture de la nervure (29),
**caractérisée**
- **en ce qu'**une pluralité de pieds de soutien (30) tournés vers le corps (15) sont formés sur la tête de piston (11), entre lesquels le corps (15) peut glisser jusqu'à ce que les pieds de soutien (30) butent contre l'extrémité du corps, et aux extrémités côté corps desquels sont rattachées des nervures (29) pelliculaires ou filiformes ou en forme de baguettes, qui réalisent la liaison avec le corps (15),
- et **en ce que** la tête de piston (11) et le corps (15) sont réalisés d'un seul tenant.

10. Seringue d'injection selon la revendication 9, **caractérisée en ce que** la tête de piston (11) est réalisée en forme de disque.
